# EUROPEAN PATENT APPLICATION

(11) **EP 1 645 221 A1**
(43) Date of publication of application: **12.04.2006**
(21) Application number: 04735507.8
(22) Date of filing: 31.05.2004
(51) Int. Cl.: A61B 3/09

(54) **INSTRUMENT FOR MEASURING EYE ADJUSTING FUNCTION STATE**

(30) Priority: 14.07.2003 JP 2003273833
(71) Applicant: RIGHT MFG. Co. Ltd., Tokyo, 174-8633 (JP)
(72) Inventor: NAGATA, Tatsuhiko, RIGHT MFG, CO., LTD., Tokyo 174-8633 (JP); AIZAWA, Eishi, RIGHT MFG, CO., LTD., Tokyo 174-8633 (JP); NISHIDA, Tetsurou, RIGHT MFG, CO., LTD., Tokyo 174-8633 (JP)
(74) Representative: Ruttensperger, Bernhard
(86) International application number: PCT/JP2004/007874
(87) International publication number: WO 2005/004708

(57) **Abstract**

The present invention provides an eye accommodation function state measurement device capable of always obtaining accurate measurement results regardless of the cylinder power, measurement date, and the like, and enabling measurement under conditions the same as the previous measurement conditions. The eye accommodation function state measurement device includes a target projection section 62 which projects a target 62a onto a subject's eye 60, and a target moving mechanism which moves the position of the target 62a along the direction of the optical axis of the subject's eye 60, the eye accommodation function state measurement device disposing the target 62a at a plurality of positions by using the target moving mechanism, and measuring the eye accommodation function state of the subject's eye 60 at each position, the eye accommodation function state measurement device including a target position select section 67 which can select the initial position of the target 62a from a plurality of positions, and a target position correction section 68 which can correct the initial position of the target 62a to an arbitrary position. The target position select section 67 can select one of at least three kinds of power values including "spherical power", "spherical power + cylinder power / 2", and "spherical power + cylinder power". as the initial position of the target 62a.

## Description

### TECHNICAL FIELD

The present invention relates to an eye refractive power measurement device which measures the refractive power of the subject's eye and the accommodation function state of the subject's eye.

### BACKGROUND ART

In the field of medical treatment such as ophthalmology, eye accommodation function state measurement has been demanded. For example, a device which objectively measures the eye accommodation function has been proposed, such as an eye accommodation function state measurement device disclosed in patent document 1.

In the eye accommodation function state measurement, according to the patent document 1, the eye's refractive power is continuously measured in the same manner as in a known refractive power measurement method (e.g. method disclosed in patent document 2), and high-frequency components of the refractive power are calculated from the measured refractive power values to determine the eye accommodation function state. The method disclosed in the patent document 1 requires continuous refractive power measurement for high-frequency components of 1 to 2.3 Hz. When the frequency is set at 1 Hz, an eye refractive power measurement section measures the refractive power at intervals of 0.1 sec, for example. The continuous measurement is performed for about 20 sec/cycle at a number of positions while moving the target position (e.g. about eight cycles at eight positions).

In known eye accommodation function state measurement, preliminary measurement is conducted before measuring the eye accommodation function state to obtain a measurement far point position (refractive power used to calculate the target position when starting measurement), and the target position is determined based on the measurement far point position.

The refractive power is measured in order to obtain the measurement far point position. In the refractive power measurement, a spherical power (generally called "sphere" (hereinafter indicated as "S")), a cylinder power (generally called "cylinder" (hereinafter indicated as "C")), and an astigmatism axis (generally called "axis" (hereinafter indicated as "Ax") are measured. In a related-art method, the measurement far point position is determined by using only the spherical power. This is because, since the eye accommodation function state measurement observes a change in the refractive power, it suffices to use only the spherical power which can be easily measured.

When the cylinder power of the subject is small, the measurement far point position can be determined by using only the spherical power. However, when the cylinder power of the subject is large, a difference occurs between the absolute far point position (hereinafter called "absolute far point"), which is one of the characteristics of a subject's eye 60, and the measurement far point position.

Moreover, since the refractive power easily changes day by day, the measurement far point position does not necessarily coincide with the absolute far point. When a difference occurs between the measurement far point position and the absolute far point position, an accurate measurement result cannot be obtained by the eye accommodation function state measurement. In the case where the absolute far point of the subject is accurately known, it is preferable to replace the measurement far point with the absolute far point.

When comparing the measurement value with the previous measurement value for a subject who has undergone measurement, the target position must be the same as that of the previous measurement. However, since the refractive power easily changes as described above, the refractive power may be measured at a different target position due to a change in the measurement far point.
(Patent document 1) JP-A-2003-70740
(Patent document 2) JP-A-6-165757

### DISCLOSURE OF THE INVENTION

An objective of the present invention is to provide an eye accommodation function state measurement device capable of always obtaining an accurate measurement result regardless of the cylinder power, measurement date, and the like, and allowing measurement to be conducted under conditions the same as the previous measurement conditions.

The present invention achieves the above objective by providing the following means. The following means is described using symbols corresponding to those used in embodiments of the present invention so that the present invention is readily understood. However, the present invention is not limited thereto.

A first invention provides an eye accommodation function state measurement device comprising a target projection section (62) which projects a target (62a) onto a subject's eye (60), and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a position correction section (68) which can correct the initial position of the target to an arbitrary position.

A second invention provides an eye accommodation function state measurement device comprising a target projection section (62) which projects a target (62a) onto a subject's eye (60), and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a position select section (67) which can select the initial position of the target from a plurality of positions.

A third invention provides the eye accommodation function state measurement device according to the second invention, wherein the position of the target (62a) is calculated from an eye's refractive power, and the position select section (67) can select the eye's refractive power used to calculate the initial position of the target from at least three kinds of power values including "spherical power", "spherical power + cylinder power / 2", and "spherical power + cylinder power".

A fourth invention provides an eye accommodation function state measurement device comprising a target projection section (62) which projects a target (62a) onto a subject's eye (60), and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a position select section (67) which can select the initial position of the target from a plurality of positions, and a position correction section (68) which can correct the position of the target selected by the position select section to an arbitrary position.

A fifth invention provides the eye accommodation function state measurement device according to the fourth invention, wherein the position of the target (62a) is calculated from an eye's refractive power, and the position select section (67) can select the eye's refractive power used to calculate the initial position of the target from at least three kinds of power values including "spherical power", "spherical power + cylinder power / 2", and "spherical power + cylinder power"..

A sixth invention provides the eye accommodation function state measurement device according to the fifth invention, wherein "spherical power", "spherical power + cylinder power / 2", or "spherical power + cylinder power" selected by the position select section (67) is used to calculate the eye accommodation function state.

A seventh invention provides an eye accommodation function state measurement device comprising a target projection section (62) which projects a target (62a) onto a subject's eye (60), and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a measurement result call section (65) which reads a previous measurement result from inside and/or outside of the eye accommodation function state measurement device.

An eighth invention provides the eye accommodation function state measurement device according to the seventh invention, comprising a display section (66) which can display the previous measurement result read by the measurement result call section (65) together with a latest measurement result.

The present invention exhibits the following effects.
(1) Since the eye accommodation function state measurement device includes the position correction section which can correct the initial position of the target to an arbitrary position or the position select section which can select the initial position of the target from a plurality of positions, accurate measurement can be conducted regardless of the cylinder power, measurement date, and the like.
(2) Since the position select section can select the initial position of the target from at least three kinds of power values including "spherical power", "spherical power + cylinder power / 2", and "spherical power + cylinder power"., accurate measurement can be more easily conducted regardless of the cylinder power, measurement date, and the like.
(3) Since "spherical power", "spherical power + cylinder power / 2", or "spherical power + cylinder power" selected by the position select section is used to calculate the eye accommodation function state, an accurate measurement result can be calculated under conditions the same as the previous measurement conditions.
(4) Since the eye accommodation function state measurement device includes the measurement result call section which reads the previous measurement result from inside and/or outside of the eye accommodation function state measurement device, the previous measurement result can be easily referred to.
(5) Since the eye accommodation function state measurement device includes the display section which can display the previous measurement result read by the measurement result call section together with the latest measurement result, the previous measurement result and the latest measurement result can be easily compared.

### BRIEF DESCRIPTION OF THE DRAWINGS

FIG 1 is a configuration diagram of an eye accommodation function state measurement device 51 according to one embodiment of the present invention.
FIG 2 is a diagram showing a striped pattern of a chopper 61a.
FIG 3 is an operation flowchart of a control section 65.
FIG 4 is a diagram showing an example in which previous data and current data are simultaneously displayed.

### BEST MODE FOR CARRYING OUT THE INVENTION

The present invention achieves the objective of always obtaining an accurate measurement result regardless of the cylinder power, measurement date, and the like, and allowing measurement to be conducted under conditions the same as the previous measurement conditions using a simple configuration by providing a position correction section which can correct the position of the target when starting the measurement (hereinafter referred to as "initial position of the target") to an arbitrary position and/or a position select section which can select the initial position of the target from a plurality of positions.

Embodiments of the present invention are described below with reference to the drawings.

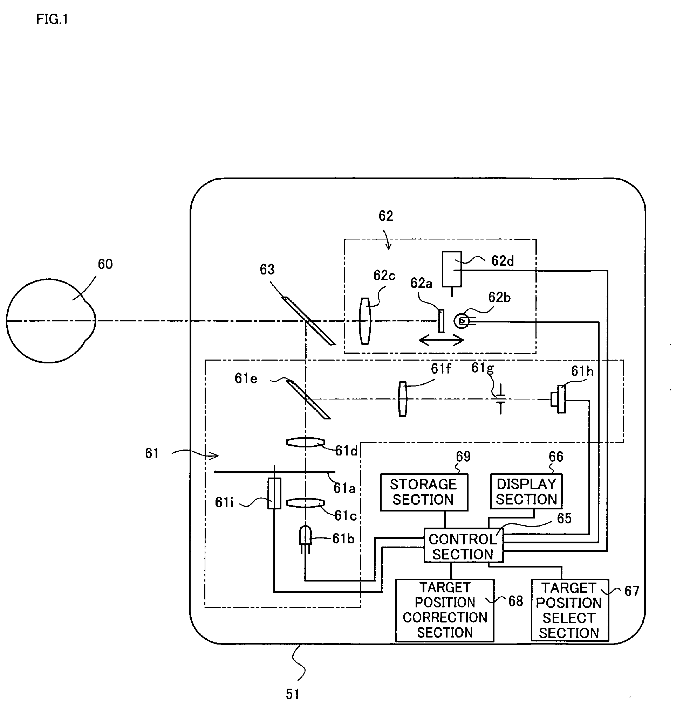

FIG 1 is a configuration diagram of an eye accommodation function state measurement device 51 according to one embodiment of the present invention. The configuration of the device used in the present invention is similar to those of the devices disclosed in the patent documents 1 and 2. The device used in the present invention utilizes retinoscopy as the measurement principle. The basic principle of obtaining one refraction measurement value is similar to that disclosed in the patent documents 1 and 2. Therefore, details of the measurement principle are omitted.

As shown in FIG 1, the eye accommodation function state measurement device 51 includes a refraction measurement section 61, a target projection section 62, a dichroic mirror 63, a control section 65, a display section 66, a target position select section 67, a target position correction section 68, a storage section 69, and the like. An external storage section 69 for storing data is provided outside the eye accommodation function state measurement device 51.

In the projection section 62, a convex lens 62c, a target 62a, and a light source 62b are disposed in the order from the subject's eye 60. A luminous flux from the target 62a illuminated by the light source 62b is incident on the subject's eye 60 after being converted into a state similar to a parallel luminous flux by the convex lens 62c. Therefore, the target 62a is seen from the subject's eye 60 at a position distant from the actual position. The target 62a and the light source 62b can be moved by using a target moving mechanism (not shown) and a motor 62d in the direction of the optical axis of the subject's eye 60 while maintaining a constant positional relationship.

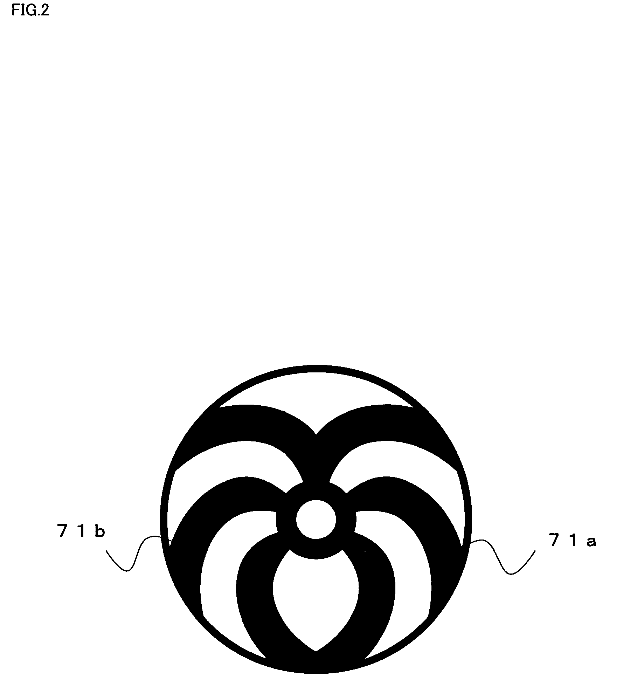

FIG 2 is a diagram showing a striped pattern of a chopper 61a.

The refraction measurement section 61 includes the chopper 61a having slits formed therein, a motor 61i which rotates the chopper 61a, a light source (infrared light source) 61b which illuminates the chopper 61, a lens 61d which projects a striped pattern formed by the chopper 61a onto the fundus of the subject's eye 60, a light-receiving section 61h which detects the moving velocity of the striped pattern formed by light returned from the fundus of the subject's eye 60, a lens 61f, a diaphragm 61g, and the like. The refraction measurement section 61 also includes a lens 61c, a half mirror 61e, and the like.

The dichroic mirror 63 respectively guides measurement light (infrared light) emitted from the refraction measurement section 61 and measurement light (visible light) emitted from the projection section 62 to the subject's eye 60, and returns the infrared light from the subject's eye 60 to the refraction measurement section 61. In the refraction measurement section 61, the chopper 61a rotates so that the striped pattern projected onto the fundus of the subject's eye 60 moves. The moving velocity of the striped pattern formed on the light-receiving section 61h changes corresponding to the refractive power of the subject's eye 60. As shown in FIG 2, stripes 71a and 71b in two directions are formed on the chopper 61a as the striped pattern. When the chopper has made a round, two meridional directions are measured so that the refractive power such as the spherical power (S), the cylinder power (C), and the astigmatic axis (Ax) are calculated.

The control section 65 includes a CPU, a circuit including a memory used for the operation of the CPU, and the like. The control section 65 controls the operations of the light sources 62b and 61b, the motors 62e and 61i, and the display section 66, and performs a calculation by referring to signals output from the light-receiving section 61h. In more detail, the control section 65 disposes the target 62a (target 62a and light source 62b) and changes (scans) the position of the target 62a by referring to the output from the refraction measurement section 61 while driving the refraction measurement section 61 (controlling the operation of the motor 62d while driving the light source 62b).

The control section 65 determines the refractive power of the subject's eye 60 as described above by referring to the output from the light-receiving section 61h while driving the light source 61b, the motor 61i, and the light-receiving section 61h.

The control section 65 stores data in the storage section 69 and reads measurement results from the storage section 69 as a measurement result call section.

A method of causing the control section 65 to perform accommodation function state measurement by using the device having the above-described configuration is described below.

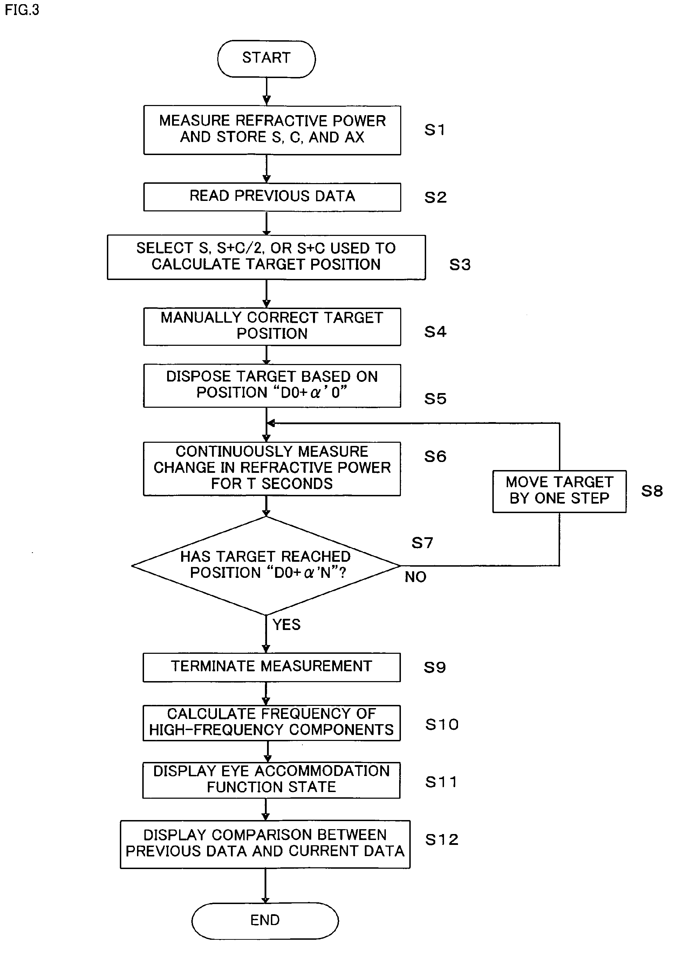

FIG 3 is an operation flowchart of the control section 65.

As preparation measurement before conducting main measurement, a far point position D0 of the subject's eye 60 is measured. The measurement far point position D0 is the target position at which the subject's eye can see the farthest target, and is measured in order to adjust the main measurement procedure corresponding to the characteristics of the subject's eye 10. The far point position is measured in the same manner as in general refractive power measurement, and the measurement method is disclosed in the patent document 1. Therefore, details of the far point position measurement are omitted. The device according to the present invention determines the measurement start target position as described below based on the measurement far point position D0.

After the refraction measurement has been completed, the spherical power (S), the cylinder power (C), and the astigmatic axis (Ax) are stored (step 1: "step" is hereinafter abbreviated as "S").

Then, the previous data of the subject is read (S2). Specifically, the control section 65 reads the subject's previous measurement data from the storage section 69. This aims at confirming in advance the target position at which the previous measured value was obtained, the presence or absence of a change in the spherical power (S), cylinder power (C), and astigmatism axis (Ax), and "spherical power (S)", "spherical power + cylinder power / 2 (S+C/2)", or "spherical power + cylinder power (S+C)" selected in the previous measurement.

Then, "spherical power (S)", "spherical power + cylinder power / 2 (S+C/2)", or "spherical power + cylinder power (S+C)" used to determine the measurement far point position used to calculate the initial position of the target 62a is selected by using the target position select section (position select section) 67 (S3). This aims at selecting "spherical power (S)", "spherical power + cylinder power / 2 (S+C/2)", or "spherical power + cylinder power (S+C)" used to obtain the previous measured data.

Moreover, even when the previous data does not exist, the visibility differs when the cylinder power (C) is large. A human tends to see a target at a position at which a blur occurs to a minimum extent. Therefore, a subject with astigmatism tends to place the far point at the position "C/2" at which a blur due to astigmatism becomes minimum. Accordingly, the position convenient for the subject can be selected by selecting "spherical power + cylinder power / 2 (S+C/2)".

In the case where the subject completely corrects astigmatism at ordinary times by using glasses or the like, since the subject is accustomed to seeing with astigmatism, a blur is reduced by selecting "spherical power + cylinder power (S+C)". These operations can be selected by a doctor by utilizing his experience.

The unit for "spherical power (S)", "spherical power + cylinder power / 2 (S+C/2)", and "spherical power + cylinder power (S+C)" is Dp (dioptar), and Dp=1/f (f is focal length (m)). For example, f is 0.5 m at 2 Dp, and f is 1 m at 1 Dp. These values indicate the power of the eye focusing on a position 0.5 m or 1 m forward. The measurement can be conducted by placing the target 62a at this position. In one embodiment of the present invention, the target position is calculated from the selected measurement far point position D0 ("spherical power (S)", "spherical power + cylinder power / 2 (S+C/2)", or "spherical power + cylinder power (S+C)") so that the target 62a is placed at a position corresponding to the calculated position through the lens 62c.

Then, the target position is manually corrected by using the target position correction section (correction section) 68 (S4). This aims at correcting the target position when the far point of the subject is accurately known or it is desired to adjust the target position to the position at which the previous data was obtained. The target position may be corrected by inputting a numerical value by using a ten-key pad or moving the target position at a predetermined step value in the positive or negative direction. The amount of correction may be set at zero when correction is unnecessary.

The main measurement procedure is then performed. Specifically, the target 12a is disposed at a position calculated based on the far point position D0 corrected by using the target position correction section (correction section) 68. The target 62a is disposed at a position a little farther than the position calculated based on the far point position D0 (position calculated based on "D0+a'0") (S5). This position (position calculated based on "D0+a'0") is the position at which the subject's eye 60 cannot clearly see the target 62a even after accommodation but the target 62a is not blurred to a large extent. The target 62a is disposed at such a position (position calculated based on "D0+a'0") in order to prevent unnecessary movement of the subject's eye 60. Therefore, it is preferable that a'0 be about 0.5 Dp.

The target 62a is continuously disposed at an identical position for a specific time T, and a time-varying refractive power change is monitored (S6). The time T (period in which time-varying refractive power change data is sampled) is about eight seconds or more and about 20 seconds or less within which the burden on the ciliary muscle due to staring of the subject's eye 60 is small. The time T is set at about eight seconds or more because it is necessary to sample a sufficient amount of data in order to maintain the accuracy of calculation (S7) for determining the occurrence frequency of high-frequency components. In the following description, the time T is set at 20 seconds.

Occurrence of ciliary spasm differs depending on the accommodation effort for seeing the target 62a. Therefore, it is preferable to respectively acquire the accommodation function state at different target positions a'1, a'2, .... In the main measurement procedure according to one embodiment of the present invention, as the target 62a moves from the position "D0+a'0" toward the subject's eye 60 in step (e.g. 0.5 Dp) units (i.e. a' is changed in the negative direction), time-varying refractive power change data for the time T is acquired at each target position (a'1, a'0, ...). The step movement and data acquisition are repeatedly performed until the position of the target 62a reaches a predetermined position "D0+a'n" sufficiently near the subject's eye 60 (a'n = -3 Dp). In order to determine whether or not the target has reached the predetermined position "D0+a'n" sufficiently near the subject's eye 60, the control section 65 determines whether or not the target has reached the predetermined position "D0+a'n" in S7. If the target has not reached the predetermined position "D0+a'n" ("NO"), the control section 65 moves the target by one step (e.g. 0.5 Dp) (S8). Then, the measurement is performed in S6 in the same manner as described above. When the control section 65 has determined that the target has reached the predetermined position "D0+a'n" ("YES"), the control section 65 terminates the measurement (S9).

The occurrence frequency of predetermined high-frequency components is then calculated as the index of the accommodation function state from the resulting time-varying refractive power change data (S10). As the refraction value used when calculating the occurrence frequency of high-frequency components, "spherical power (S)", "spherical power + cylinder power / 2 (S+C/2)", or "spherical power + cylinder power (S+C)" selected in S3 is also used.

Then, the eye accommodation function state is displayed (S11). In this case, the results of the previous data and the current data are displayed on the screen to facilitate comparison between the previous data and the current data (S12).

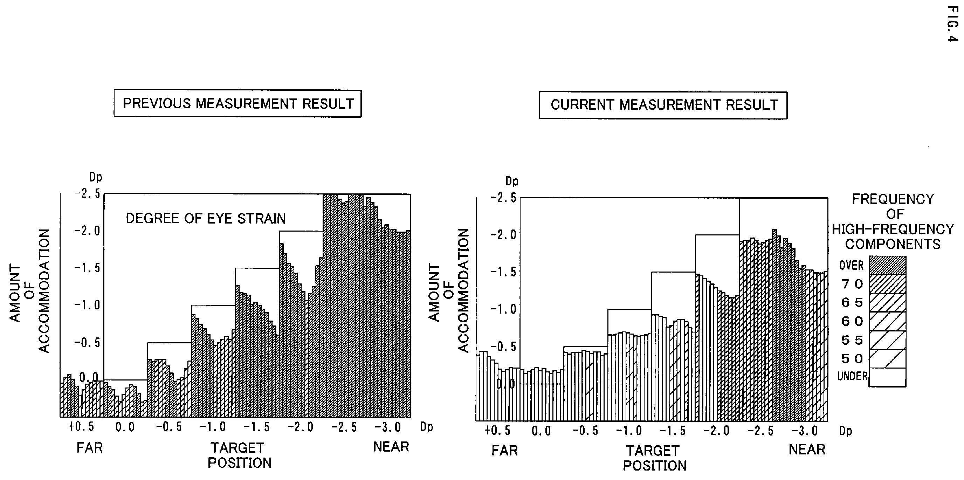

FIG. 4 is a diagram showing an example in which the previous data and the current data are simultaneously displayed.

In FIG 4, the previous measurement results are displayed on the left, and the current measurement results are displayed on the right.

The eye accommodation function state is displayed in a dark color when the amount of accommodation is large, and is displayed in a light color when the amount of accommodation is small. In FIG 4, the depth of the color is indicated by the density of hatching lines. By comparing the depth of the color between the right and left (previous data and current data), the effect of treatment, the degree of improvement, or the degree of worsening can be easily determined. In the example shown in FIG 4, the color is dark on the left (previous data) and is light on the right (current data) so that it is easily understood that the eye accommodation state has been improved by the treatment.

The calculation method for the high-frequency components and the display method for the eye accommodation function state measurement are essentially the same as those described in the patent document 1. Therefore, description of these methods is omitted.

According to one embodiment of the present invention, it is possible to take astigmatism into consideration when calculating the measurement far point position.

Moreover, it is possible to adjust the measurement conditions to the previous measurement conditions or adjust the far point to the subject's absolute far point by enabling correction of the target position, so that accurate measurement can be conducted.

Furthermore, the degree of improvement or worsening of the eye accommodation state of the subject can be easily determined by displaying the previous measured values and the current measured values side by side.

The present invention is not limited to the above-described embodiments, and various modifications and variations may be made. Such modifications and variations are also within the scope of equivalence of the present invention.
(1) The above-described embodiments illustrate an example in which the target position is selected and adjusted at the beginning of measurement. However, the target position may not necessarily be selected and adjusted for each subject. For example, when the subject has not undergone measurement and an accurate far point value is not known, selection or adjustment of the target position may be omitted.
(2) In the above-described embodiments, the storage section 69 is included in the device. However, an external storage section such as a personal computer may be used when the amount of data is large.
(3) In the above-described embodiments, only one piece of previous measurement data is displayed. However, when two or more pieces of previous measurement data exist, one or more pieces of selected previous measurement data may be displayed, or the previous measurement data may be displayed at the same time.
(4) In the above-described embodiments, the previous data and the current data are displayed in the display section of the device. However, when using a personal computer or the like as an external storage section, data may be similarly displayed in a display section of the personal computer or the like.

## Claims

1. An eye accommodation function state measurement device comprising a target projection section which projects a target onto a subject's eye, and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a position correction section which can correct the initial position of the target to an arbitrary position.

2. An eye accommodation function state measurement device comprising a target projection section which projects a target onto a subject's eye, and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a position select section which can select the initial position of the target from a plurality of positions.

3. The eye accommodation function state measurement device according to claim 2, wherein the position of the target is calculated from an eye's refractive power, and the position select section can select the eye's refractive power used to calculate the initial position of the target from at least three kinds of power values including "spherical power", "spherical power + cylinder power / 2", and "spherical power + cylinder power"..

4. An eye accommodation function state measurement device comprising a target projection section which projects a target onto a subject's eye, and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a position select section which can select the initial position of the target from a plurality of positions, and a position correction section which can correct the position of the target selected by the position select section to an arbitrary position.

5. The eye accommodation function state measurement device according to claim 4, wherein the position of the target is calculated from an eye's refractive power, and the position select section can select the eye's refractive power used to calculate the initial position of the target from at least three kinds of power values including "spherical power", "spherical power + cylinder power / 2", and "spherical power + cylinder power"..

6. The eye accommodation function state measurement device according to claim 5, wherein "spherical power", "spherical power + cylinder power / 2", or "spherical power + cylinder power" selected by the position select section is used to calculate the eye accommodation function state.

7. An eye accommodation function state measurement device comprising a target projection section which projects a target onto a subject's eye, and a target moving mechanism which moves a position of the target along a direction of an optical axis of the subject's eye, the eye accommodation function state measurement device disposing the target at a plurality of positions by using the target moving mechanism, and measuring an accommodation function state of the subject's eye at each position, the eye accommodation function state measurement device including a measurement result call section which reads a previous measurement result from inside and/or outside of the eye accommodation function state measurement device.

8. The eye accommodation function state measurement device according to claim 7, comprising a display section which can display the previous measurement result read by the measurement result call section together with a latest measurement result.
